# EUROPEAN PATENT SPECIFICATION

(11) **EP 1 778 324 B1**
(45) Date of publication and mention of the grant of the patent: **07.10.2009**
(21) Application number: 05754302.7
(22) Date of filing: 27.06.2005
(51) Int. Cl.: A61M 16/00, A61M 16/18

(54) **DEVICE FOR RECOVERING ANAESTHETIC DURING USE OF INHALED ANAESTHETICS**
VORRICHTUNG ZUR WIEDERGEWINNUNG VON ANÄSTHETIKUM IM GEBRAUCH VON INHALIERTEN ANÄSTHETIKA
DISPOSITIF DESTINE A LA RECUPERATION D'UN ANESTHESIQUE DURANT L'UTILISATION D'ANESTHESIQUES INHALES

(30) Priority: 19.07.2004 SE 0401892
(43) Date of publication of application: 02.05.2007
(73) Proprietor: Sedana Medical AB, 174 57 Sundbyberg (SE)
(72) Inventor: LAMBERT, Hans, S-113 51 Stockholm (SE)
(74) Representative: Axelsson, Nils Ake A.L.
(86) International application number: PCT/SE2005/001010
(87) International publication number: WO 2006/009498

(56) References cited:
- EP-A1- 0 761 249
- EP-A1- 0 945 151
- EP-A2- 0 911 052
- EP-A2- 1 222 940
- DE-A1- 4 326 556
- US-A- 5 471 979
- US-A- 5 509 405
- US-A- 6 155 255
- US-B1- 6 206 002

## Description

### Technical Field

The present invention relates to a device for recovering anaesthetic during use of inhaled anaesthetics. The device is connectable to a patient via a patient tube, and comprises absorption means for absorbing and desorbing anaesthetic preparations and measuring means for measuring the patient's uptake of anaesthetic, wherein a supply means for supplying anaesthetic preparations is connected to the patient tube between said absorption means and the patient. The supply means comprises an anaesthetic vaporizer arranged in the patient tube and a supply unit for supplying liquid anaesthetic preparations to said anaesthetic vaporizer, said supply unit being connectable to a container holding anaesthetic preparations in liquid phase.

### Background of the Invention

Such devices are disclosed in EP B1-0 855 924, EP-B1-0 944 407, and SE-B-495 155, for example. The purpose of such a device is to reduce the consumption of anaesthetic preparations when treating a patient, since preparations such as halothane (2-bromine-2-chlorine-1,1,1-trifluorethane) are often relatively expensive. According to older, conventional technology the anaesthetic preparation is conducted away to the surroundings together with the air exhaled and is therefore lost, as well as contaminating the surroundings.

The device of EP-B1-0 944 407 is additionally equipped with a heat and moisture exchanger (HME) in series with the absorption means for absorbing and desorbing anaesthetic preparations, the HME being capable of absorbing and desorbing water vapour, in order to compensate the loss of moisture upon exhalation.

It is vital to control the patient's uptake of anaesthetic in order to keep the patient in a stable condition under anaesthetic treatment. Generally, this is achieved by monitoring the concentration of anaesthetic in the respiratory gas of the patient and maintaining said concentration at a prescribed suitable level. However, the patient's uptake of anaesthetic can change during the anaesthetic treatment due to changes in respiratory frequency, e.g., and therefore it is necessary to continuously control the patient's uptake of anaesthetic, and depending on this control increase or decrease the input of anaesthetic to the respiratory gas inhaled by the patient in order to keep the concentration of anaesthetic at a prescribed level.

EP-B1-0 496 336 discloses an apparatus for the administration of a respiratory gas and at least one anaesthetic to a patient. The apparatus comprises an anaesthetic vaporizer which contains liquid anaesthetic, a regulating valve for controlling the flow of gas through the anaesthetic vaporizer, and an inlet tube which conducts the respiratory gas and the vaporized anaesthetic to the patient. Further, the device comprises a regulating device for control of the regulating valve so that the gas passing through the anaesthetic vaporizer attains a flow which, when saturated with anaesthetic, correlates to a preselected concentration of anaesthetic in the respiratory gas supplied to the patient. The device can also comprise a sensor for measuring the concentration of the anaesthetic in the respiratory gas, located between the anaesthetic vaporizer and the patient, the sensor being connected to the regulating device, and the regulating device controls the flow of respiratory gas depending on the measured concentration of anaesthetic.

However, the solution suggested in EP-B1-0 496 336 is complicated and makes it difficult to keep the concentration of anaesthetic in the respiratory gas stably at a prescribed level suitable for the patient.

EP-A-0 761 249 discloses an anaesthetic apparatus with a dosing unit for an anaesthetic agent. The dosing unit pumps out anaesthetic in droplet form onto a vaporizer and comprises several containers holding a liquid anaesthetic agent connectable to a micropump, and a control unit controls the micropump and regulates the dispensing of liquid anaesthetic.

### The Object of the Invention

The object of the present invention is to provide a device for recovering anaesthetic during use of inhaled anaesthetics, which in an uncomplicated manner controls the patient's uptake of anaesthetic, and more specifically, in a more stable manner, keeps the concentration of anaesthetic in the respiratory gas inhaled by the patient tube at a prescribed level.

### Summary of the Invention

The above mentioned object is achieved by providing a device as defined in the appended claim 1. By the control device, which controls the supply of liquid anaesthetic preparations to the anaesthetic vaporizer based on measurements of the patient's uptake of anaesthetic, a device for recovering anaesthetic is provided, which keeps the concentration of anaesthetic in the respiratory gas in the patient tube at a prescribed level in a less complicated and more stable manner compared to known devices, and thus in an uncomplicated manner controls the patient's uptake of anaesthetic.

According to an advantageous embodiment of the device according to the present invention, the control device is arranged to control the supply unit for control of the rate of the supply of liquid anaesthetic preparations to the anaesthetic vaporizer. The rate of the supply is the amount of liquid anaesthetic preparations supplied per unit of time. The supply can be both continuous and intermittent. If the supply is intermittent, the rate also involves the time periods between the supply doses, and the size of the doses.

According to other advantageous embodiments of the device according to the present invention, said supply unit comprises a syringe pump arranged to pump liquid anaesthetic preparations from said container to the anaesthetic vaporizer, or a suction pump arranged to suck liquid anaesthetic preparations from said container for further transport to the anaesthetic vaporizer, or an eccentric pump arranged to pump liquid anaesthetic preparations from said container to the anaesthetic vaporizer.

According to yet other advantageous embodiments of the device according to the present invention, the measuring means comprise a second measuring unit for measuring the heart rate and/or the blood pressure and/or various brain functions and/or various heart functions of the patient. Examples of various brain functions and various heart functions known in prior art are Bispectral Index (BIS), ENTROPY, and Auditory Evoked Potential (AEP), e.g.

According to another advantageous embodiment of the device according to the present invention, the device comprises a storing means for storing physiological parameters of the patient, to which the control device is connected, and that the control device is arranged to control the supply unit, for control of the supply of liquid anaesthetic preparations to the anaesthetic vaporizer, based on the physiological parameters stored in the storing means. For example, the storing means is arranged to store any of the physiological parameters body weight, height, age, and gender of the patient.

### Brief Description of the Drawings

The present invention will now be described, for exemplary purposes, in more detail by way of one embodiment and with reference to the enclosed drawings, in which:
- Fig. 1: is a schematic view of a device for recovering anaesthetic during use of inhaled anaesthetics according to known technology, and
- Fig. 2: is a schematic view of an embodiment of the device for recovering anaesthetic during use of inhaled anaesthetics according to the present invention.

### Detailed Description of a Preferred Embodiment

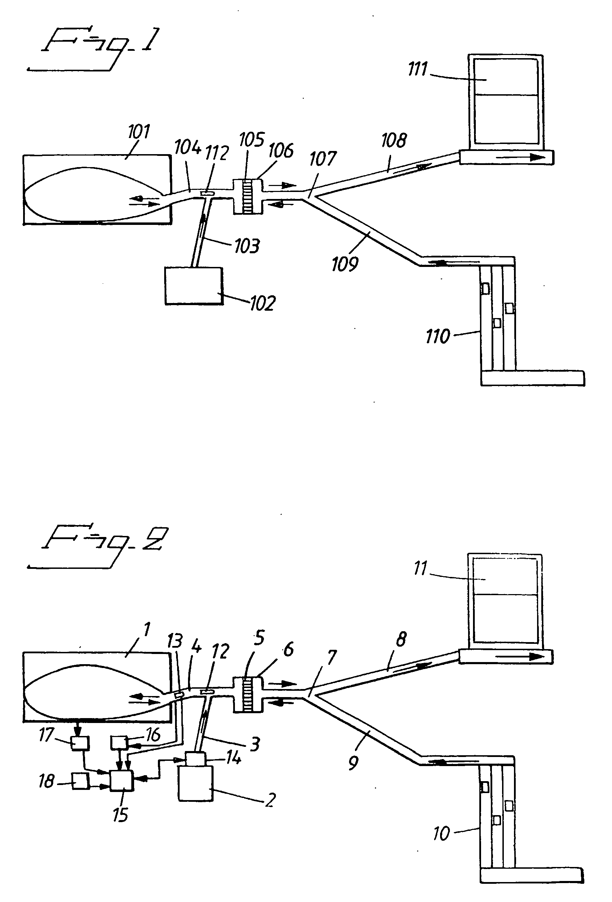

In Fig. 1, which illustrates the principle for recovering anaesthetic during use of inhaled anaesthetics according to known technology, 101 denotes a patient being treated with anaesthetic or an apparatus simulating the respiratory organs of a patient. The patient 101 is connected via a patient tube 104 to absorption means 105 for absorbing and desorbing anaesthetic preparations, a so-called anaesthetic reflector, arranged in a housing 106. A supply tube 103 for liquid anaesthetic, connected to a container 102 holding anaesthetic preparations in liquid phase, opens into the patient tube 104. The side of the absorption means 105 facing away from the patient 101 is connected via a Y-connection 107 to an outlet tube 108 for exhaled gas and an inlet tube 109 for inhaled gas, these being connected to a gas pump 111 and a gas supply device 110, respectively. An anaesthetic vaporizer 112 is arranged in the patient tube 104 where the supplied liquid anaesthetic is vaporized, and is designed according to known technology.

The absorption means 105 consists of a material having the ability to absorb or desorb anaesthetic, e.g. woven active carbon. Measurements indicate that such a device is able to return about 70 % of the anaesthetic exhaled.

The device according to the present invention illustrated in an embodiment in Fig. 2 is constructed in an equivalent manner to that shown in Fig. 1, with the following additional features. A gas monitor 13 is arranged in the patient tube 4 for measuring the concentration of anaesthetic in the respiratory gas. A supply unit 14 in the form of a suction pump is connected to the container 2 holding anaesthetic preparations in liquid phase, and is arranged to suck liquid anaesthetic preparations from said container 2 for further transport via the supply tube 3 to the anaesthetic vaporizer 12. A control device 15 is connected to the gas monitor 13 and the supply unit 14, and is arranged to control the supply unit 14, for control of the rate of the supply of liquid anaesthetic preparations to the anaesthetic vaporizer 12, based on input from the gas monitor 13. Further, a first measuring unit 16 for measuring the respiratory frequency and/or the tidal volume of the patient 1, and a second measuring unit 17 for measuring the heart rate, the blood pressure, various brain functions, and various heart functions of the patient 1 are connected to the control device 15, and the control device 15 is arranged to control the supply unit 14, for control of the rate of the supply of liquid anaesthetic preparations to the anaesthetic vaporizer 12, based on the input from said measuring units 16, 17. Finally, a storing means 18 for storing physiological parameters of the patient 1, e.g. body weight, height, age, and gender of the patient is connected to the control device 15, and the control device 15 is arranged to control the supply unit 14, for control of the rate of the supply of liquid anaesthetic preparations to the anaesthetic vaporizer 12, based on the physiological parameters stored in the storing means 18. The control device is arranged to control the supply unit 14 in order to keep the concentration of anaesthetic in the respiratory gas in the patient tube 4 at a prescribed level, which can be predetermined and/or constant.

## Claims

1. A device for recovering anaesthetic during use of inhaled anaesthetics, said device being connectable to a patient (1) via a patient tube (4), and comprising absorption means (5) for absorbing and desorbing anaesthetic preparations and measuring means for measuring the patient's (1) uptake of anaesthetic which comprise a gas monitor (13) arranged in the patient tube (4) for measuring the concentration of anaesthetic in the respiratory gas, wherein a supply means for supplying anaesthetic preparations is connected to the patient tube (4) between said absorption means (5) and the patient (1), said supply means comprising an anaesthetic vaporizer (12) arranged in the patient tube (4) and a supply unit (14) for supplying liquid anaesthetic preparations to said anaesthetic vaporizer (12), said supply unit (14) being connectable to a container (2) holding anaesthetic preparations in liquid phase, wherein the measuring means also comprise a first measuring unit (16) for measuring the respiratory frequency and/or the tidal volume of the patient (1), and the device comprises a control device (15) connected to the measuring means and the supply unit (14), which control device (15) is arranged to control the supply unit (14), for control of the supply of liquid anaesthetic preparations to the anaesthetic vaporizer (12), based on input from the measuring means, to keep the concentration of anaesthetic in the respiratory gas in the patient tube (4) at a prescribed level.

2. A device according to claim 1, **characterized in that** the control device (15) is arranged to control the supply unit (14) for control of the rate of the supply of liquid anaesthetic preparations to the anaesthetic vaporizer (12).

3. A device according to claim 1 or 2, **characterized in that** said supply unit (14) comprises a syringe pump arranged to pump liquid anaesthetic preparations from said container (2) to the anaesthetic vaporizer (12).

4. A device according to claim 1 or 2, **characterized in that** said supply unit (14) comprises a suction pump arranged to suck liquid anaesthetic preparations from said container (2) for further transport to the anaesthetic vaporizer (12).

5. A device according to claim 1 or 2, **characterized in that** said supply unit (14) comprises an eccentric pump arranged to pump liquid anaesthetic preparations from said container (2) to the anaesthetic vaporizer (12).

6. A device according to any of the claims 1-5, **characterized in that** the measuring means comprise a second measuring unit (17) for measuring the heart rate and/or the blood pressure and/or various brain functions and/or various heart functions of the patient (1).

7. A device according to any of the claims 1-6, **characterized in that** the device comprises a storing means (18) for storing physiological parameters of the patient (1), to which the control device (15) is connected, and that the control device (15) is arranged to control the supply unit (14), for control of the supply of liquid anaesthetic preparations to the anaesthetic vaporizer (12), based on the physiological parameters stored in the storing means (18).

8. A device according to claim 7, **characterized in that** the storing means (18) is arranged to store any of the physiological parameters body weight, height, age, and gender of the patient (1).

## Patentansprüche

1. Vorrichtung zum Zurückgewinnen von Anästhetikum während der Verwendung von inhalierten Anästhetika, wobei die Vorrichtung über eine Patientenleitung (4) mit einem Patienten (1) verbindbar ist und Absorptionsmittel (5) zum Absorbieren und Desorbieren von anästhetischen Präparaten und Messmittel zum Messen der Aufnahme von Anästhetikum durch den Patienten (1) umfaßt, die ein Gaskontrollgerät (13) umfassen, das in der Patientenleitung (4) angeordnet ist, um die Konzentration von Anästhetikum in dem Atemgas zu messen, wobei ein Zufuhrmittel zum Zuführen von anästhetischen Präparaten mit der Patientenleitung (4) zwischen den Absorptionsmitteln (5) und dem Patienten (1) verbunden ist, wobei das Zufuhrmittel einen anästhetischen Verdampfer (12), der in der Patientenleitung (4) angeordnet ist, und eine Zufuhreinheit (14) zum Zuführen von flüssigen anästhetischen Präparaten zu dem anästhetischen Verdampfer (12) umfasst, wobei die Zufuhreinheit (14) mit einem Behälter (2) verbindbar ist, der anästhetische Präparate, in flüssigem Zustand beinhaltet, wobei die Messmittel auch eine erste Messeinheit (16) zum Messen der Atemfrequenz und/oder des Atemvolumens des Patienten (1) umfassen, und die Vorrichtung eine Steuerungsvorrichtung (15) aufweist, die mit den Messmitteln und der Zufuhreinheit (14) verbunden ist, welche Steuervorrichtung (15) angeordnet ist, um die Zufuhreinheit (14) zur Steuerung der Zufuhr von flüssigen anästhetischen Präparaten zu dem anästhetischen Verdampfer (12) auf der Grundlage von Eingaben von den Messmitteln zu steuern, um die Konzentration von Anästhetikum in dem Atemgas in der Patientenleitung (4) auf einem vorgeschriebenen Niveau zu halten.

2. Vorrichtung nach Anspruch 1, **dadurch gekennzeichnet**, das die Steuerungsvorrichtung (15) angeordnet ist, um die Zufuhreinheit (14) zum Steuern der Zufuhrrate von flüssigen anästhetischen Präparaten zu dem anästhetischen Verdampfer (12) zu steuern.

3. Vorrichtung nach Anspruch 1 oder 2, **dadurch gekennzeichnet**e dass die Zufuhreinheit (14) eine Spritzpumpe aufweist, die angeordnet ist, um flüssige anästhetische Präparate aus dem Behälter (2) zu dem anästhetischen Verdampfer (12) zu pumpen.

4. Vorrichtung nach Anspruch 1 oder 2, **dadurch gekennzeichnet**, das die Zufuhreinheit (14) eine Saugpumpe umfasst, die angeordnet ist, um flüssige anästhetische Präparate aus dem Behälter (2) zum weiteren Transport zu dem anästhetischen Verdampfer (12) zu saugen.

5. Vorrichtung nach Anspruch 1, oder 2, **dadurch gekennzeichnet**, das die Zuführeinheit (14) eine Exzenterpumpe umfasst, die angeordnet ist, um flüssige anästhetische Präparate aus dem Behälter (2) zu dem anästhetischen Verdampfer (12) zu pumpen.

6. Vorrichtung nach einem der Ansprüche 1 bis 5, **dadurch gekennzeichnet, dass** die Messmittel eine zweite Messeinheit (17) zum Messen der Herzfrequenz und/oder des Blutdrucks und/oder verschiedener Hirnfunktionen und/oder verschiedener Herzfunktionen des Patienten (1) umfassen.

7. Vorrichtung nach einem der Ansprüche 1 bis 6, **dadurch gekennzeichnet, dass** die Vorrichtung ein Speichermittel (18) zum Speichern von physiologischen Parametern des Patienten (1) umfasst, mit dem die Steuerungsvorrichtung (15) verbunden ist, und dass die Steuerungsvorrichtung (15) angeordnet ist, um die Zufuhreinheit (14) zur Steuerung der Zufuhr von flüssigen anästhetischen Präparaten zu dem anästhetischen Verdampfer (12) auf der Grundlage der physiologischen Parameter, die in dem Speichermittel (18) gespeichert sind, zu steuern,

8. Vorrichtung nach Anspruch 7, **dadurch gekennzeichnet, dass** das Speichermittel (13) angeordnet ist, um irgendeinen der physiologischen Parameter Körpergewicht, Größe, Alter und Geschlecht des Patienten (1) zu speichern.

## Revendications

1. Dispositif pour récupérer un anesthésique pendant l'utilisation d'anesthésiques inhalés, ledit dispositif pouvant être raccordé à un patient (1) via un tube de patient (4), et comprenant des moyens d'absorption (5) pour absorber et désorber des préparations d'anesthésique et des moyens de mesure pour mesurer la prise d'anesthésique du patient (1) qui comprend un moniteur de gaz (13) agencé sur le tube de patient (4) pour mesurer la concentration d'anesthésique dans le gaz respiratoire, dans lequel des moyens d'alimentation pour alimenter les préparations d'anesthésique sont raccordés au tube de patient (4) entre lesdits moyens d'absorption (5) et le patient (1), lesdits moyens d'alimentation comprenant un vaporisateur d'anesthésique (12) agencé dans le tube de patient (4) et une unité d'alimentation (14) pour alimenter les préparations d'anesthésique liquide audit vaporisateur d'anesthésique (12), ladite unité d'alimentation (14) pouvant être raccordée à un récipient (2) contenant des préparations d'anesthésique en phase liquide, dans lequel les moyens de mesure comprennent également une première unité de mesure (16) pour mesurer la fréquence respiratoire et/ou le volume courant du patient (1) et le dispositif comprend un dispositif de commande (15) raccordé aux moyens de mesure et à l'unité d'alimentation (14), lequel dispositif de commande (15) est agencé pour commander l'unité d'alimentation (14) pour le contrôle de l'alimentation des préparations d'anesthésique liquide au vaporisateur d'anesthésique (12), basé sur l'entrée par les moyens de mesure pour maintenir la concentration d'anesthésique dans le gaz respiratoire dans le tube de patient (4) à un niveau prédéterminé.

2. Dispositif selon la revendication 1, **caractérisé en ce que** le dispositif de commande (15) est agencé pour contrôler l'unité d'alimentation (14) pour le contrôle de la vitesse d'alimentation des préparations d'anesthésique liquide au vaporisateur d'anesthésique (12).

3. Dispositif selon la revendication 1 ou 2, **caractérisé en ce que** ladite unité d'alimentation (14) comprend une pompe de seringue agencée pour pomper les préparations d'anesthésique liquide dudit récipient (2) au vaporisateur (12).

4. Dispositif selon la revendication 1 ou 2, **caractérisé en ce que** ladite unité d'alimentation (14) comprend une pompe d'aspiration agencée pour aspirer les préparations d'anesthésique liquide dudit récipient (2) pour le transport supplémentaire vers le vaporisateur d'anesthésique (12).

5. Dispositif selon la revendication 1 ou 2, **caractérisé en ce que** l'unité d'alimentation (14) comprend une pompe excentrique agencée pour pomper les préparations d'anesthésique liquide dudit récipient (2) au vaporisateur d'anesthésique (12).

6. Dispositif selon l'une quelconque des revendications 1 à 5, **caractérisé en ce que** les moyens de mesure comprennent une seconde unité de mesure (17) pour mesurer le pouls et/ou la pression du sang et/ou les différentes fonctions du cerveau et/ou les différentes fonctions du coeur du patient (1),

7. Dispositif selon l'une quelconque des revendications 1 à 6, **caractérisé en ce que** le dispositif comprend des moyens de stockage (18) pour stocker des paramètres physiologiques du patient (1) auquel le dispositif de commande (15) est raccordé, et **en ce que** le dispositif de commande (15) est agencé pour commander l'unité d'alimentation (14) pour le contrôle de l'alimentation des préparations d'anesthésique liquide au vaporisateur d'anesthésique (12), basé sur les paramètres physiologiques stockés dans les moyens de stockage (18).

8. Dispositif selon la revendication 7, **caractérisé en ce que** les moyens de stockage (18) sont agencés pour stocker l'un quelconque des paramètres physiologiques tels que le poids, la taille, l'âge et le sexe du patient (1).
